# EUROPEAN PATENT SPECIFICATION

(11) **EP 0 690 912 B1**
(45) Date of publication and mention of the grant of the patent: **05.03.2008**
(21) Application number: 94912411.9
(22) Date of filing: 14.04.1994
(51) Int. Cl.: C12N 15/86, C12N 7/01, C12N 15/33, A61K 39/235, C07K 14/08

(54) **RECOMBINANT AVIAN ADENOVIRUS VECTOR**
REKOMBINANTER ADENOVIRUSVEKTOR FÜR GEFLÜGEL
VECTEUR D'ADENOVIRUS DE RECOMBINAISON AVIEN

(30) Priority: 14.04.1993 AU PL829793
(43) Date of publication of application: 10.01.1996
(60) Divisional application: 05076351.5
(73) Proprietor: COMMONWEALTH SCIENTIFIC AND INDUSTRIAL RESEARCH ORGANISATION, Campbell, ACT 2601 (AU)
(72) Inventor: SHEPPARD, Michael, Lincoln, NE 68516 (US); ERNY, Katrina, CH-1010 Lausanne (CH); McCOY, Richard, North Balwyn, VIC 3104 (AU); WERNER, Wendy, Burwood, VIC 3125 (AU)
(74) Representative: Maschio, Antonio
(86) International application number: PCT/AU1994/000189
(87) International publication number: WO 1994/024268

(56) References cited:
- EP-A2- 0 156 478
- WO-A-91/16925
- WO-A-93/03145
- AU-B- 562 548
- AU-B- 576 907
- DEVELOPMENTAL BIOLOGY, vol. 166, no. 1, 1994, pages 355-359, XP000607522 YAMAGATA, M. ET AL.: "Gene transfer to avian embryo with a recombinant adenovirus"
- Am. J. Vet. Res., Vol. 52, No. 7 (1991), pages 1137-1141, ZHANG C. et al., "Identification and Characterization of Viral Polypeptides from Type-II Avian Adenoviruses". XP000607599
- Virology, 188 (1992), pages 881-886, SHEPPARD M. and TRIST H., "Characterization of the Avian Adenovirus Penton Base". XP000608409

## Description

### FIELD OF INVENTION

This invention relates to delivery vectors for antigen producing genes (heterologous gene sequences) used to generate immune responses in commercial poulty flocks susceptible to decimation by disease. Such vectors are especially useful for the preparation of vaccines which can be easily administered on a large scale to protect poultry flocks against disease. This invention also relates to a method of production of suitable delivery vectors, to methods of preparation of vaccines based on the vectors, to administration strategies and to a method of protecting poultry from disease.

### BACKGROUND

The productivity of the intensive poultry industry depends on the control of infectious diseases. In Australia the cost of disease to industry is conservatively estimated at $50 million annually. Whilst diseases can be controlled in part by good hygiene and quarantine measures, the industry must still rely on vaccination to protect flocks. In a commercial situation the difficulty and cost in the administration of suitable preventative or curative agents or adjuvants is caused by the sheer number of poultry to be treated. Thus vaccines must be cheap, effective and easy to deliver.

Conventionally, vaccines constituting live viral particles have been prepared by virus passage and selection of attenuated forms. Alternatively killed vaccines were prepared from virulent viruses.

One recent attempt to vaccinate commercial bird flocks against a common viral infection is that described in AU-A-34353/93 (VIROGENETICS CORPORATION). This application describes a recombinant poxvirus such as vaccinia virus or fowlpox virus containing heterologous DNA from the Marek's disease virus which is used as a vaccine to induce an immunological response in a host animal. However, the use of poxviruses has the disadvantage that the immune response of poultry is not sustained sufficiently long enough to generate adequate protection. Particularly, the immunoprotection generated may not be sufficient to protect a bird once maternal antibody has ceased to play a role in antibody mediated immunity.

A different approach has been taken by a group at Medisorb Technologies, Inc., in the U.S. as reported in the journal Genetic Engineering News, September 1993. This approach used a conventional Salmonella vaccine encapsulated in a biodegradable microsphere based on polylactic-coglycolic acid. The approach requires, however that the microsphere be injected into the bird. This approach is not necessarily commercially feasible for large flocks.

It is thus an aim of this invention to provide a delivery vehicle for heterologous sequences of genetic material that is particularly suited to administration on a large scale.

In particular, it is an aim of this invention to provide or enhance means for generation and/or optimisation of antibodies or cell-mediated immunity so as to provide protection against infection with common avian diseases, which means is quickly and effectively administered, particularly to large flocks of poultry. It is an additional aim to provide a process for preparation of a suitable means for generation and/or optimisation of antibodies or cell-mediated immunity so as to protect birds against infection with common avian diseases. It is a further aim to provide a protection strategy.

### SUMMARY OF INVENTION

The invention provides, in one embodiment, a recombinant avian adenovirus vector incorporating, and capable of expression of, at least one heterologous nucleotide sequence, wherein the at least one heterologous nucleotide sequence is inserted into a non-essential region at the right terminal end of the genome of the avian adenovirus.

Preferably the heterologous nucleotide sequence is capable of expression as an antigenic polypeptide.

The antigenic polypeptide encoded by the at least one nucleotide sequence is preferably foreign to the host vector.

The recombinant vector may comprise a live recombinant avian adenovirus in which the virion structural proteins are unchanged from those in the native avian adenovirus from which the recombinant avian adenovirus is produced.

The invention is predicated upon the discovery that certain regions of the Fowl Adenovirus have unique properties. In particular, the major late promoter and leader sequences are quite dissimilar to equivalent regions in Adenoviruses previously characterised. It has surprisingly been discovered that the leader sequence is a dipartite leader sequence. It is also surprising, based on knowledge of human adenoviruses that there are non-essential regions in the fowl Adenovirus genome which do not correspond to those characterised previously in other Adenoviruses thus making this virus particularly suited to delivery of heterologous sequences.

This invention is further predicated on the discovery that the Avian adenovirus generates a prolonged response in poultry thus making it well suited as a vaccine vehicle. Furthermore, the existence of a number of serotypes of varying virulence allows the selection of a vaccine vehicle suited to the level of immune response required.

Adenoviruses are a large and diverse family, having been isolated from many living species, including man and other mammals, as well as a variety of birds, particularly chickens (Wigand, R., Gelderblom, H. and Ozell, M. Biological and Biophysical characteristics of mouse adenovirus, strain FL. Arch, Virol. 54: 131-142; 1977). As a result adenoviruses have been separated into two different genera, one group has a mammalian host range *(Mastadenoviradae)* and the other an avian host range *(Aviadenoviradae).* Because the avian adenovirus serotypes are only very distantly related to mammalian adenoviruses a knowledge of the latter is only partially instructive in relation to the former. The avian adenoviruses show only very limited DNA homology with human adenoviruses (Alestrom, P., Steniund, A., Li, P., Bellet, A.J.D. and Pettersson, U. Sequence homology between avian and human adenoviruses. J. Virol. 42: 306-310; 1982) with fowl adenovirus ("FAV") genomes being some 10 kilobases larger than the human adenovirus genome. The classification of these viruses as adenoviruses is based solely on morphological and structural similarities.

The genus Aviadenovirus is currently divided into 5 species groups; fowl, turkey, goose, pheasant and duck adenoviruses. Fowl adenoviruses were first recognized in the 1950s when they were isolated as a consequence of using embryonated eggs and cell cultures infected with FAV (Van Den Ende, M., Don, P.A. and Kipps, A. The isolation in eggs of a new filterable agent which may be the cause of bovine lumpy skin disease. J. gen. Micro. 3: 174-182; 1949; Yates, V.J. and Fry, D.E. Observations on a chicken embryo lethal orphan (CELO) virus (serotype 1). Am. J.Vet. Res. 18: 657-660; 1957). However, the only fowl adenovirus upon which some molecular study has been undertaken is the oncogenic FAV, chicken embryo lethal orphan (CELO). The CELO virus genome is almost 30% longer than that of human adenoviruses (HAV) (Laver, W.G., Bandfield-Younghusband, H. and Wrigley, N,G. Purification and properties of chick embryo lethal orphan virus (an avian adenovirus). Virol. 45: 598-614; 1971). CELO virus is composed of at least 11-14 structural proteins (Yasue, H. and Ishibashi, M. Chick embryo lethal orphan (CELO) virus-induced early and late polypeptides. Virol. 78:216-233; 1977; Li, P., Bellet, A.J.D. and Parish, C.R. DNA-binding proteins of chick embryo lethal orphan virus: Lack of complementation between early proteins of avian and human adenoviruses. J. gen. Virol. 65: 1817-1825; 1984a) and is structurally similar to HAV which is composed of 10-14 structural proteins (Maizel, J.V. (Jr.), White, D.O. and Scharff, M. The polypeptides of adenovirus. I. Evidence for multiple protein components in the virion and a comparison of types 2, 7A and 12. Virol. 36: 115-125; 1968; Ishibashi, M. and Maizel, (Jr.), J.V. The polypeptides of adenovirus V. Young virions, structural intermediate between top components and aged virions. Virol. 57:409-424; 1974). The only morphological difference apparent between them is the additional FAV fibre. Even allowing for a second fibre gene a considerable amount of 'excess' DNA remains to be accounted for in the genome of FAV when compared to the HAV.

DNA cross-hybridization studies demonstrated only very limited homology between HAV and FAV (Alestrom, P., Stenlund, A., Li, P., Bellet, A.J.D. and Pettersson, U. Sequence homology between avian and human adenoviruses. J. Virol. 42: 306-310; 1982). However, in spite of this and the lack of immunological relatedness, the amino acid composition of CELO virus and HAV hexons but not the gene sequence were found to be similar (Laver, W.G., Bandfield-Younghusband, H. and Wrigley, N,G. Purification and properties of chick embryo lethal orphan virus (an avian adenovirus). Virol. 45: 598-614; 1971). Other similarities noted include; the presence of terminal repeat sequences at either end of the genome (Alestrom, P., Stenlund, A., Li, P., Bellet, A.J.D. and Pettersson, U. Sequence homology between avian and human adenoviruses. J. Virol. 42: 306-310; 1982; Sheppard, M. and Erny, K.M. DNA sequence analysis of the inverted terminal repeats of a non-oncogenic avian adenovirus. Nuc. Acids Res. 17: 3995; 1989), the synthesis of low molecular weight virus associated ("VA") RNAs (Larsson, S., Bellet, A. and Akusjarvi, G. VA RNAs from avian and human adenoviruses: dramatic differences in length, sequence, and gene location. J.Virol. 58: 600-609; 1986) and the sharing of at least one non-structural protein; the DNA binding protein (DBP) (Li, P., Bellet, A.J.D. and Parish, C.R. DNA-binding proteins of chick embryo lethal orphan virus: Lack of complementation between early proteins of avian and human adenoviruses. J. gen. Virol. 65: 1817-1825; 1984a).

At face value these findings suggest strong similarities exist between FAV and HAV. However the terminal repeat sequences of FAV are much shorter in length and, unlike HAV, the length of terminal repeats did not differ in a comparison between one oncogenic and one non-oncogenic FAV (Sheppard, M. and Erny, K.M. DNA sequence analysis of the inverted terminal repeats of a non-oncogenic avian adenovirus. Nuc. Acids Res. 17: 3995; 1989). Secondly, the VA RNA genes of FAV differ from their HAV counterparts in their chromosomal location, direction of transcription and primary sequence (Larsson, S., Bellet, A. and Akusjarvi, G. VA RNAs from avian and human adenoviruses: dramatic differences in length, sequence, and gene location. J.Virol. 58: 600-609; 1986). Finally the DBP of FAV which initiates transcription by interacting with the E1A genes, fails to recognize the E1A genes of HAV (Li, P., Bellet, A.J.D. and Parish, C.R. DNA-binding proteins of chick embryo lethal orphan virus: Lack of complementation between early proteins of avian and human adenoviruses. J. gen. Virol. 65: 1817-1825; 1984a).

Fowl adenoviruses are common within poultry flocks, and to date 11 distinct serotypes have been recognized (McFerran, J.B. and Connor, T.J. Further studies on the classification of fowl adenoviruses. Av. Dis. 21:585-595; 1977). While all of these serotypes appear to be widely disseminated throughout the world, epidemiological surveys show that in a given geographical location certain serotypes appear to predominate. For example, in America the most common serotypes encountered are 1,4,5,7 and 9 (Cowen , B., Mitchell, G.B. and Calnek, B.W. An adenovirus survey of poultry flocks during the growing and laying periods. Av. Dis. 22:115-121; 1978; Yates, V.J., Rhee, Y.O., Fry, D.E., El Mishad, A.M. and McCormick, K.J. The presence of avian adenoviruses and adeno-associated viruses in healthy chickens. Av. Dis. 20: 146-152; 1976). Surveys of clinically diseased Australian flocks have identified types 1 and 4 (Boyle, D.B. and McFerran, J.B. Avian adenoviruses isolated from poultry in Queensland. Aus. Vet. J. 52: 587-589; 1976) and, more recently types 6, 7 and 8 (Reece, R.L., Barr, D.A., Grix, D.C., Forsyth, W.M., Condron, R.J. and Hindmarsh, M. Observations on naturally occurring inclusion body hepatitis in Victorian chickens. Aust.Vet. J. 63: 201-202; 1986) as the most prevalent serotypes.

When choosing appropriate FAV for development as live vectors to deliver vaccines to bird flocks, it is important to take into account the natural prevalence of serotypes. Those serotypes not commonly encountered in the field have an obvious advantage over those to which flocks are frequently exposed and to which they may have developed immunity.

A further consideration is the ability of the vector to remain active in the bird beyond the period within which maternal antibodies protect the bird immediately post-hatching.

Other important considerations in choosing potential FAV vectors are pathogenicity and immunogenicity. Preferably live vector viruses should be highly infectious but non-pathogenic (or at least stably attenuated) such that they do not themselves adversely affect the target species.

The oncogenic nature of serotype-1 FAV has been demonstrated (Sarma, P.S., Huebner, R.J. and Lane, W.T. Induction of tumours in hamsters with an avian adenovirus (CELO). Science 149: 1108; 1965) and for this reason this group of FAV is not favoured for vector development.

Preferred candidates for use as vaccine vectors are non-pathogenic isolates FAV CFA20 (serotype 10), and CFA15 (serotype 10). The CFA20 virus is the more preferred vector candidate because it is clinically safe and because it represents a serotype apparently uncommon in Australian and American poultry flocks. This is an important consideration as it reduces the possible problems associated with prior exposure. FAV CFA15 and CFA19 (serotype 9) are also suitable candidates worthy of consideration for vector development. Other serotypes may also be useful. It is notable that more virulent strains produce a greater antibody response.

Heterologous nucleotide sequences which may be incorporated into non-essential regions of the viral genome and which may encode the antigenic determinants of infectious organisms against which the generation of antibodies or cell-mediated immunity is desirable may be those expressing antigenic determinants of intestinal infections caused by parasites; for example, coccidial infections or respiratory viruses, for example, infectious bronchitis virus. Other infectious organisms against which immunity may be desirable include those that target internal organs such as the bursa of Fabricius, for example, infectious bursal disease virus (IBDV).

Heterologous nucleotide sequences which may be incorporated include the antigenic determinants of the agents of
Newcastle Disease
Marek's Disease
Egg Drop Syndrome
Inclusion Body Hepatitis
Infectious Laryngotracheitis
Mycoplasma
Chicken Anaemia Agent (aplastic anaemia)
Avian Influenza
Avian Encephalomyelitis.

Heterologous nucleotide sequences more preferred for incorporation in the vectors of the invention are those expressing antigenic determinants of infectious bursal disease (VP2) and coccidiosis.

It is also envisaged the heterologous sequences incorporated may be immunopotentiator molecules such as cytokines or growth promoters, for example chicken myelomonocytic growth factor (cMGF) or insulin like growth factors (IGF).

The type of immune response stimulated by candidate vectors may affect the selection of heterologous nucleotide sequences for insertion therein. FAV isolates CFA20 and CFA15 may induce mucosal immunity and are thus more suitable for infections of the intestines or respiratory system. FAV isolates such as CFA19 which induce a strong serum antibody response may be more suitable for use against diseases of the organs of poultry because of their ability to penetrate beyond the gut of the bird.

The DNA of interest which may comprise heterologous genes coding for antigenic determinants or immuno potentiator molecules are located in at least one non-essential region of the viral genome.

Non-essential regions of the viral genome which are suitable for the purposes of replacement with or insertion of heterologous nucleotide sequences are non-coding regions at the right terminal end of the viral genome. Preferably this region is located at the right end of the genome at map units 97 to 99.9.

The heterologous gene sequence may be associated with a promoter and leader sequence in order that the nucleotide sequence may be expressed in situ as efficiently as possible. Preferably the heterologous gene sequence is associated with the avian adenoviral major late promoter and splice leader sequence. The major late promoter lies near 16-17 map units on the adenovirus genetic map and contains a classical TATA sequence motif. (Johnson, D.C., Ghosh-Chondhury, G., Smiley, J.R., Fallis, L. and Graham, F.L, (1988). Abundant expression of herpes simplex virus glycoprotein gB using an adenovirus vector. Virology 164, 1-14).

The splice leader sequence of the avian adenovirus isolates under consideration is a dipartite sequence spliced to late genes.

The heterologous gene sequence may also be associated with a poly adenylation sequence.

Instead of the avian adenoviral major late promoter, any other suitable eukaryotic promoter can be used.

For example, those of SV40 virus, cytomegalovirus (CMV) or human adenovirus may be used.

Processing and poly adenylation signals other than those of avian adenoviruses may also be considered, for example, that of SV40.

In a further aspect of the invention there is provided a recombinant vaccine for generating and/or optimising antibodies or cell mediated immunity so as to provide or enhance protection against infection by an infectious organism in birds, said vaccine comprising at least one recombinant avian adenovirus vector incorporating, and capable of expression of at least one heterologous nucleotide sequence, the said heterologous nucleotide sequence being inserted into a non-essential region at the right terminal end of the genome of the avian adenovirus, and suitable carriers and/or excipients. Preferably the nucleotide sequence is capable of expression as an antigenic polypeptide.

The antigenic polypeptide encoded by the at least one nucleotide sequence is preferably foreign to the host vector. At least one nucleotide sequence may be associated with a promoter/leader and a poly A sequence.

The recombinant vaccine may include live recombinant avian adenovirus vector in which the virion structural protein are unchanged from that in the native avian adenovirus from which the recombinant avian adenovirus is produced.

Preferred vector candidates for use in the recombinant vaccine are FAV isolates CFA20 (serotype 10), CFA15 (serotype 10) and CFA19 (serotype 9). Use of other serotypes is possible, depending on the poultry type, its existing immunity and its environment.

The vaccine may be directed against respiratory and intestinal infections caused by a variety of agents. In order to direct the vaccine against a specific infectious organism, heterologous gene sequences encoding the antigenic determinants of those infectious organisms are incorporated into non-essential regions of the genome of the avian adenovirus comprising the vector. If the vaccine is to be used to optimise protection against disease, suitable heterologous nucleotide sequences may be those of immunopotentiators such as cytokines or growth promoters.

The vaccines may comprise other constituents, such as stabilisers, excipients, other pharmaceutically acceptable compounds or any other antigen or part thereof. The vaccine may be in the form of a lyophilised preparation or as a suspension, all of which are common in the field of vaccine production.

A suitable carrier for such a vaccine may be isotonic buffered saline.

The form of administration may be that of an enteric coated dosage unit, an inoculum for intra-peritoneal, intramuscular or subcutaneous administration, an aerosol spray, by intraocular drop or intranasal application. Administration in the drinking water, in feed pellets or in ovo is also possible.

The more preferred mode of administration is as an aerosol spray.

In another aspect of the invention, there is provided a method of producing a recombinant avian adenovirus vector for use as a vaccine comprising inserting into a non-essential region at the right terminal end of an avian adenovirus genome, at least one heterologous nucleotide sequence in association with an effective promoter sequence. Said heterologous nucleotide sequence is preferably capable of expression as an antigenic polypeptide.

Preferably the antigenic polypeptide encoded by the at least one nucleotide sequence is foreign to the host vector.

More preferably the heterologous nucleotide sequence is associated with promoter/leader and poly A sequences.

In one preferred method of construction of a vaccine vector, a restriction enzyme site preferably being one that does not cleave the host genome selected for construction, is inserted into a non-essential and preferably non-coding region of the host genome. The recombinant viral genome thus is provided with a unique restriction enzyme site in a non-essential region to allow insertion of heterologous nucleotide sequences by simple restriction enzyme cleavage and ligation. This method has the added advantage of enabling, if preferred, deletion of portions of the non-essential region to allow the insertion of greater portions of DNA.

By this method a DNA expression cassette containing an appropriate FAV promoter with a foreign gene sequence as well as leader sequences and poly adenylation recognition sequences can be constructed with the unique restriction enzyme sites flanking the cassette enabling easy insertion into the FAV genome.

In an alternative method of construction of a suitable vector the non-essential region to be altered to incorporate foreign DNA could be constructed via homologous recombination. By this method the non-essential region is cloned, a portion of it is deleted and foreign DNA together with promoter, leader and poly adenylation sequences is inserted preferably by homologous recombination between flanking sequences. By this method also, deletion of portions of the non-essential region is possible to create extra room for larger DNA inserts that are beyond the normal packaging constraints of the virus.

The vaccine is preferably administered by aerosol spray since airborne spread is a natural route of FAV dissemination.

FAV vector based vaccines may be administered as 'cocktails' comprising 2 or more virus vectors carrying different foreign genes or immunopotentiators.

In a preferred vaccination strategy, the 'cocktail' or simultaneous strategy, a vaccine based on both FAV isolates CFA19 and CFA20 is used.

In an alternative strategy, FAV vector based vaccines may be administered consecutively of each other to either administer booster vaccines or new vaccines at some stage subsequent to initial FAV vaccination. The vaccines used are preferably based on heterologous FAV isolates.

In a preferred version of the "consecutive" strategy, vaccines based on isolates serotypically unrelated are selected so as to achieve maximum protection against infection. In one example of such a strategy a vaccine based on FAV isolate CFA20 is administered subsequently or prior to vaccination with a vaccine based on FAV isolate CFA19.

Poultry are conveniently inoculated with vector vaccines according to the invention at any age. Where chickens are concerned, broilers may be vaccinated at 1 day old, breeders and layers may be vaccinated regularly up to point of lay and thereafter.

Preferably according to either the consecutive strategy or the cocktail strategy, poultry are vaccinated while still not fully immunocompetent. More conveniently, day-old birds can be vaccinated for protection against re-infection after a period of 4 weeks subsequent to initial vaccination.

An immune response can be produced in a bird by administering to the bird an effective amount of a recombinant vaccine according to the invention. An effective amount as referred to throughout the present description is an amount sufficient to elicit an immune response, preferably at least 10⁴ TCID₅₀ per dose, but within the range of 10³ - 10⁷ TCID₅₀ per dose depending on the method of application.

The vaccine of the invention may of course be combined with vaccines against other viruses or organisms such as Marek's Disease virus, Newcastle Disease virus or infectious bronchitis at the time of its administration.

In a preferred aspect of this embodiment of the invention, administration is by aerosol spray.

Methods for construction and testing of recombinant vectors and vaccines according to this invention will be well known to those skilled in the art. Standard procedures for endonuclease digestion, ligation and electrophoresis were carried out in accordance with the manufacturer's or supplier's instructions. Standard techniques are not described in detail and will be well understood by persons skilled in the art.

### PREFERRED EMBODIMENTS

Aspects of preferred embodiments of the invention based on FAV isolates CFA15, 19 and 20 will now be described. Whilst these three isolates have been selected because of their low pathogenicity and high immunogenicity, it will be appreciated that other isolates of avian adenovirus may also be suitable for construction of vaccine vectors provided that the criteria for selection described hereinbefore are met.

Table 1 illustrates the suitability of several other isolates of varying serotype. Pathogenicity was tested by administration of 10⁶ pfu of virus injected in a 0.5 ml volume via the intraperitoneal route. Surviving chickens were killed at 8-10 days and tissue samples taken for histology and virus re-isolation.

### CHARACTERISATION OF VIRAL GENOMES

**Table 1. Pathogenicity of 15 FAV representing 8 distinct and 1 intermediate serotype, for day-old chickens Injected via the intraperitoneal (IP) route.**

| Virus | Serotype | Mortalities^{a} % | Virus recovery^{b} | % Weight reduction^{c} |
|---|---|---|---|---|
| CFA3 | 8 | 0 | + | 0 |
| CFA13 | 6 | 0 | + | 8 |
| C FA20 | 10 | 0 | + | 8 |
| CFA15 | 10 | 0 | + | 0 |
| CFA2 | 1 | 5 | + | 0 |
| CFA7 | 1 | 5 | + | 0 |
| CFA11 | 2 | 10 | + | 9 |
| CFA17 | 8 | 15 | + | 1 |
| CFA19 | 9 | 18 | + | 17 |
| CFA10 | 7 | 20 | + | 16 |
| CFA4 | 7 | 35 | + | 25 |
| CFA9 | 1 | 50 | + | 25 |
| CFA5 | 8 | 65 | + | 25 |
| CFA22 | 7/8 | 100 | + | ₋d |
| CFA24 | 7/8 | 100 | + | ₋d |
| a- twenty, day-old chickens each received 10⁶ pfu of virus and subsequent deaths were recorded over an 8 day period | | | | |
| b- virus recovery was attempted from caecal tonsil tissue | | | | |
| c- weight reduction was calculated by comparing the average weight of infected birds with that of uninfected control birds | | | | |
| d- None survived long enough to determine weight reduction | | | | |

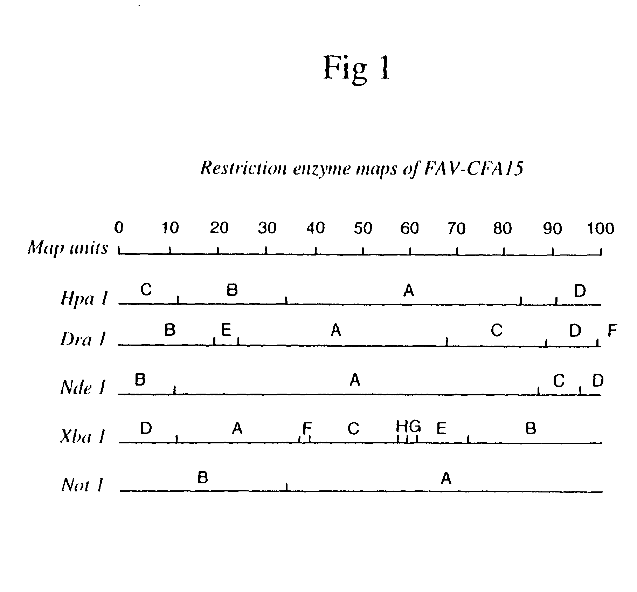

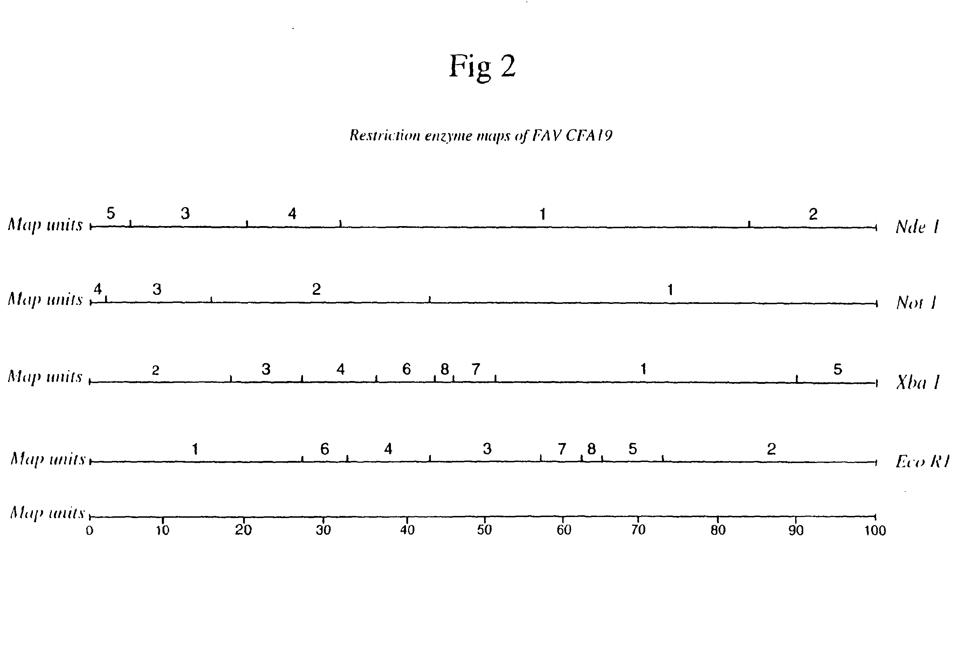

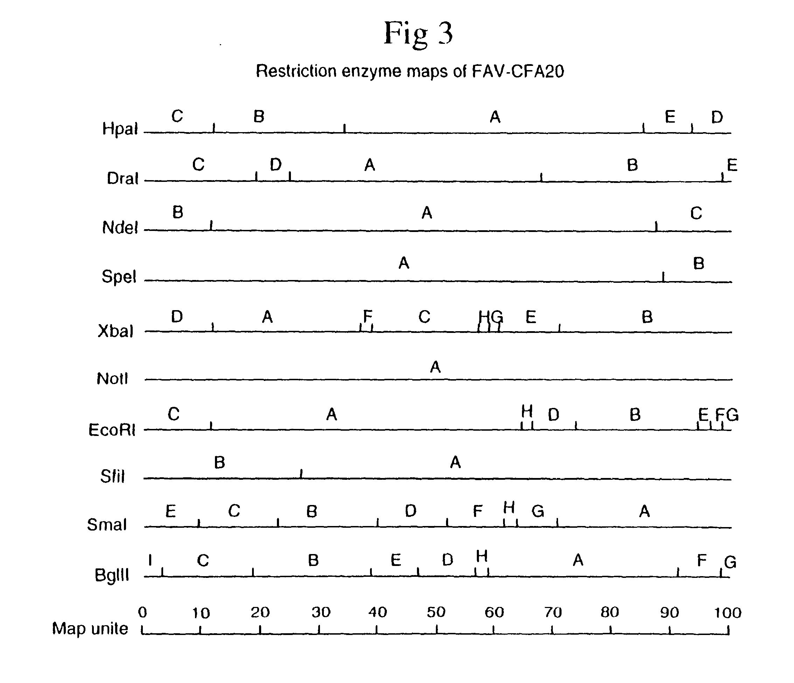

The genomes of the selected isolates FAV CFA15, 19 and 20 were characterised by conventional methods. The DNA restriction endonuclease maps of the entire genome of each are illustrated respectively in figures 1, 2 and 3. Map units are given (1 m.u = 0.45Kb) and the genomes are oriented left to right. By convention adenovirus genomes are normally oriented such that the terminal region from which no late mRNA transcripts are synthesised is located at the left end. The enzyme used to generate each map is indicated at the edge of each map.

### CHARACTERISATION OF MAJOR LATE PROMOTER (MLP) AND SPLICE LEADER SEQUENCES (LS) OF CFA20

### Identification and cloning of the FAV MLP

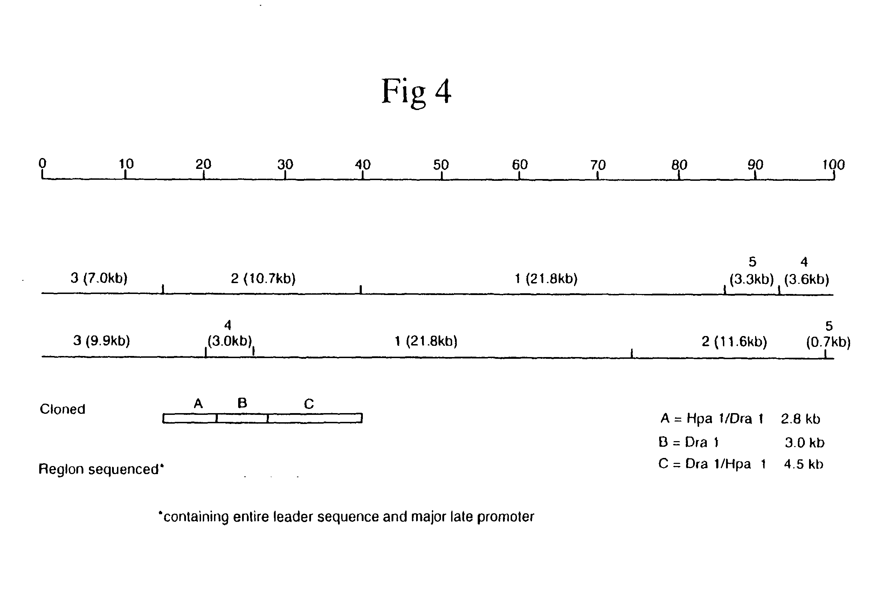

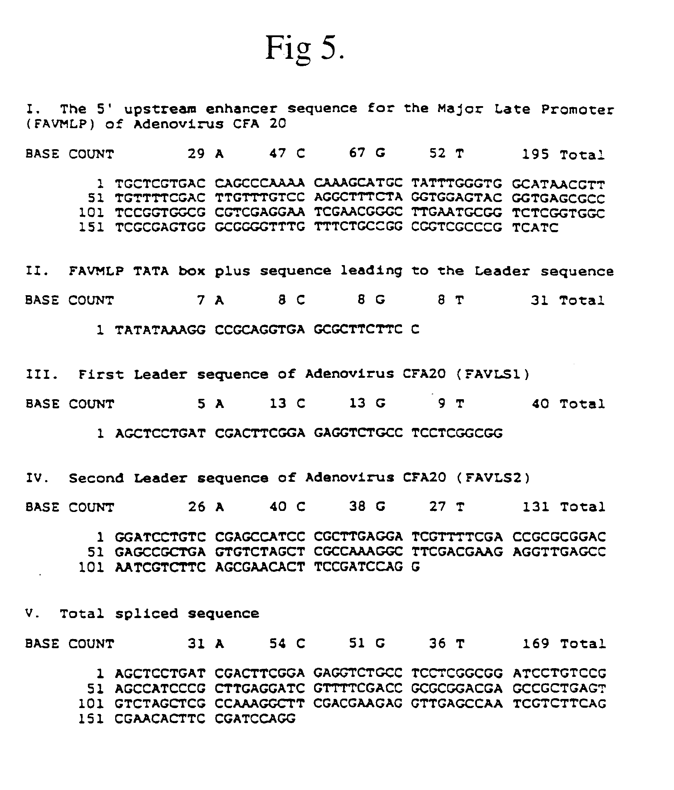

By use of DNA restriction enzyme and genetic maps of the FAV serotype 10 (CFA20) genome, a region was located that was believed to contain the MLP and leader sequences. Three DNA fragments *DraI* fragment 4 (3.0kb), *Hpal*/*Dral* fragment (2.8kb) and a *DraI*/*HpaI* fragment (4.5kb) (Fig. 4) were all cloned individually into plasmid vectors. These DNAs were subcloned into M13mp18 and mp19 and sequenced (Fig. 5).

The MLP promoter sequence was identified as containing a classical TATA sequence, the only one in the region sequenced, as well as potential upstream factors and was subsequently confirmed by the location of the leader sequence and the transcriptional start site.

Figure 4 illustrates the sequence characterisation and cloning of the major late promoter and splice leader sequences of CFA20. Specifically, shown are the *HpaI* and *DraI* restriction endonuclease maps of FAV CFA20. The regions cloned (▭) and sequenced (-) are indicated.

In order to determine the structure and sequence of the leader sequence spliced to late mRNA, chicken kidney cells were infected with FAV and the infection was allowed to proceed until late in the infection cycle (usually 24-32 hr p.i.). At this time total RNA was purified from the infected cells using RNAzol B solution (Bresatec, Australia). The isolated RNA was precipitated with isopropanol and stored at -70°C in 50 µl aliquots until required. Poly A (mRNA) was isolated from total RNA by the use of the Poly AT tract System (Promega, USA). The isolated mRNA was used in cDNA production.

For cDNA production, oligonucleotides were produced to the complementary strand of the hexon gene and the penton base gene, both being MLP transcripts. A further oligo was produced which covered the proposed cap site of the major late transcript, 24 bases downstream of the TATA box. This oligonucleotide was used in conjunction with that used in cDNA production in Taq polymerase chain reaction. The resulting DNA produced from positive clones was digested with appropriate restriction enzymes to determine the size of the inserted fragment. DNA sequencing of these inserted fragments was performed using a modification of the chain termination technique (Sanger, F., Nicklen, S. and Gulson, A.R. (1977) DNA sequencing with chain terminating inhibitors PNAS USA 74: 5463-5467) so as to allow T7 DNA polymerase extension (Pharmacia, Sweden).

To confirm the leader sequence cap site, fresh cDNA was prepared and this time a tail of dGTP residues added to it. Briefly, cDNA was incubated with 1 mM dGTP and approximately 15 units of terminal deoxynucleotidyl transferase (Pharmacia) in 2 mM CaCl₂ buffer at 37°C for 60 minutes. The reaction was stopped by heating to 70°C for 10 minutes. The DNA was then ethanol precipitated and resuspended in a volume suitable for use in polymerase chain reaction (PCR). PCR was performed as previously described using a poly (dC) oligonucleotide with a *XbaI* site at the 5' end. Resulting fragments were digested with *XbaI* and *SmaI* and cloned into pUCI9 vector. DNA preparation and sequencing were performed, as described previously, on clones shown to be positive by hybridization.

Figure 5 illustrates the DNA sequence of the major late promoter, upstream enhancer sequences and splice leaders 1 and 2 showing the arrangement of splice leader 1 from cDNA studies.

### CHARACTERISATION OF NON-ESSENTIAL REGIONS OF VIRAL GENOME

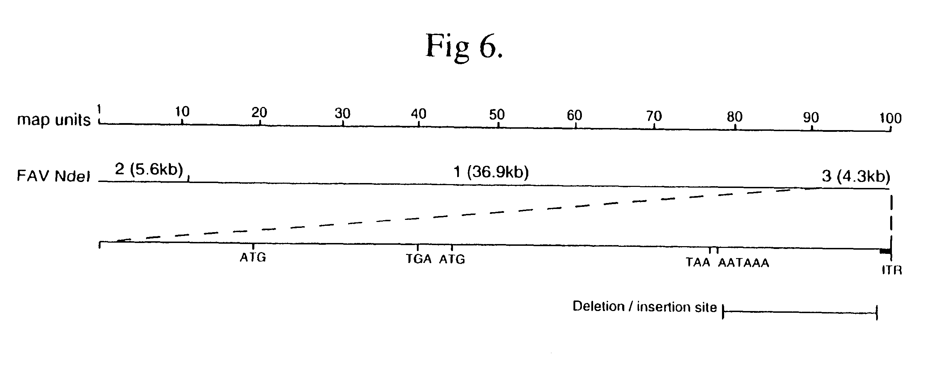

The right end was identified by cloning and complete sequencing of the FAV (serotype 10 CFA 20) *Ndel*/*3* fragment of 4249 bp. The entire organisation of genes in the right end (contained in the *Ndel*/*3* fragment) of FAV appears to be very different to other adenoviruses that have been characterised. This region contains two open reading frames terminating by base 3361 (Fig. 6). This leaves a relatively large region of 824 bp that is available for deletion and insertion of foreign DNA. This will allow at least 3.1 map units of previously unidentified and unexpected DNA for deletion and therefore insertion.

Figure 6 illustrates an expanded region from 92-100 map units for the FAV CFA20 genome and shows the non-coding region available for deletion of virus DNA and insertion of heterologous DNA. Also indicated are possible coding regions.

### CONSTRUCTION OF FAV VECTOR

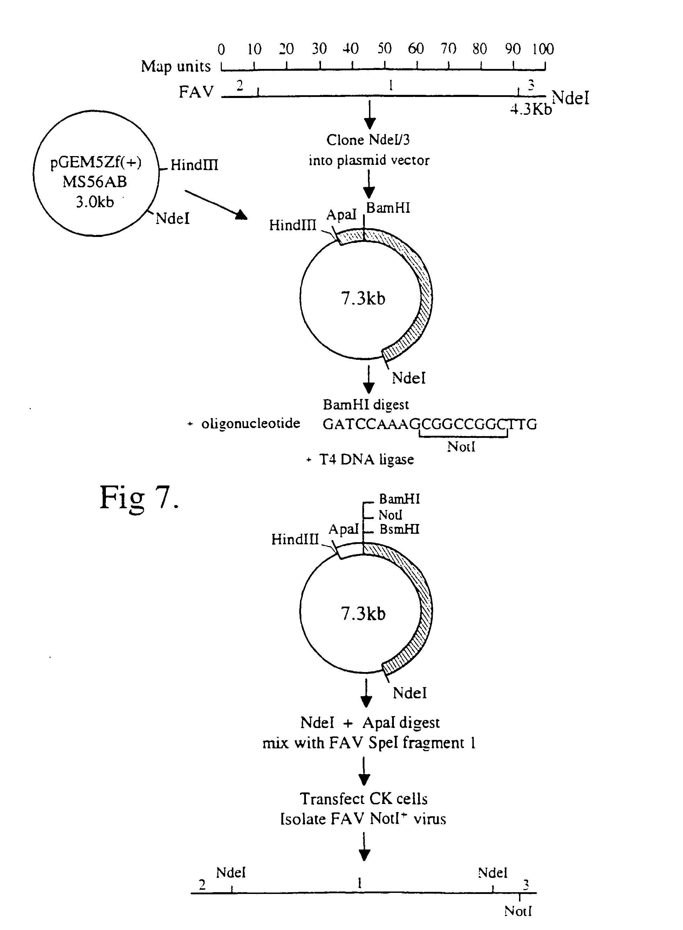

Figure 7 illustrates a preferred method of construction of a FAV vector. The right hand end *NdeI* fragment 3 of the FAV CFA20 is cloned and a unique restriction endonuclease *(NotI)* site is inserted. CK cells were transfected with purified altered *NdeI* fragment 3 and purified *SpeI* fragment 1 to produce a functional virus by homologous recombination with a unique restriction endonuclease *(NotI)* site in the genome. One particularly preferred FAV isolate is that identified as FAV M11 *NotI* which was deposited at the Australian Government Analytical Laboratories on 11 March, 1994 and given the accession number N94/8879.

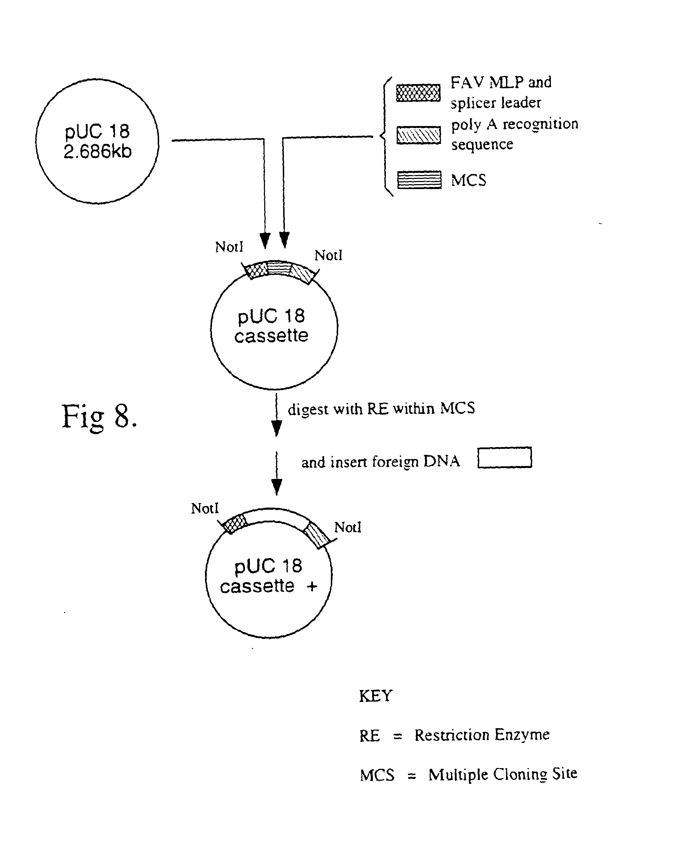

Figure 8 illustrates construction of an expression cassette for FAV. The major late promoter and splice leader sequences 1 and 2 are inserted into a plasmid vector as well as multiple cloning sites for insertion of foreign DNA and a poly A recognition site. All these are flanked by unique restriction endonuclease recognition sequences *(NotI)* for insertion into the unique restriction endonuclease site in the FAV genome.

### Example of vector construction

### 1. Construction of FAV-vp22 recombinant

The IBDV vp2 gene was inserted into the expression cassette as described in Figure 8. The expression cassette was isolated as a *NotI* fragment and cloned into the unique *NotI* site previously engineered into the FAV *NdeI*/*3* fragment. The plasmid containing the vp2 gene was linearised and transfected together with FAV *SpeI*/1 DNA into CK cells. This plasmid was deposited at the Australian Government Analytical Laboratories on 11 March 1994 and can be identified in the bacterium E.coli DH52 pFMLP 234. This bacterium has been given the accession number N94/8878.

### 2. Construction of a FAV thymidine kinase recombinant

The Infectious Laryngotracheitis Virus (ILTV) thymidine kinase (TK) gene was inserted into an expression cassette, as described in Figure 8, utilising the CMVIE promoter and SV40 polyadenylation signal to regulate expression. The TK expression cassette was isolated as a *NotI* fragment (Figure 8) and cloned into the unique *NotI* site previously engineered into the FAV *NdeIl3* fragment (Figure 7). The plasmid containing the TK expression cassette, flanked by the FAV *NdeI*/3 fragment, was linearised by digestion with *NdeI* and transfected, together with FAV genomic DNA, into CK cells. FAV vectors expressing the TK gene were selected by passage through methatrexate, a metabolic inhibitor that blocks the replication of TK deficient (wild type) viruses. Southern blot hybridisation, following plaque purification, confirmed the presence of the TK gene within the *NdeI*/*3* fragment of the resulting FAV vector. The ability to isolate a FAV-TK recombinant by passage through methatrexate, clearly demonstrates the potential to insert, and express, foreign genes within this region of the FAV genome.

### EXPRESSION OF FOREIGN GENES

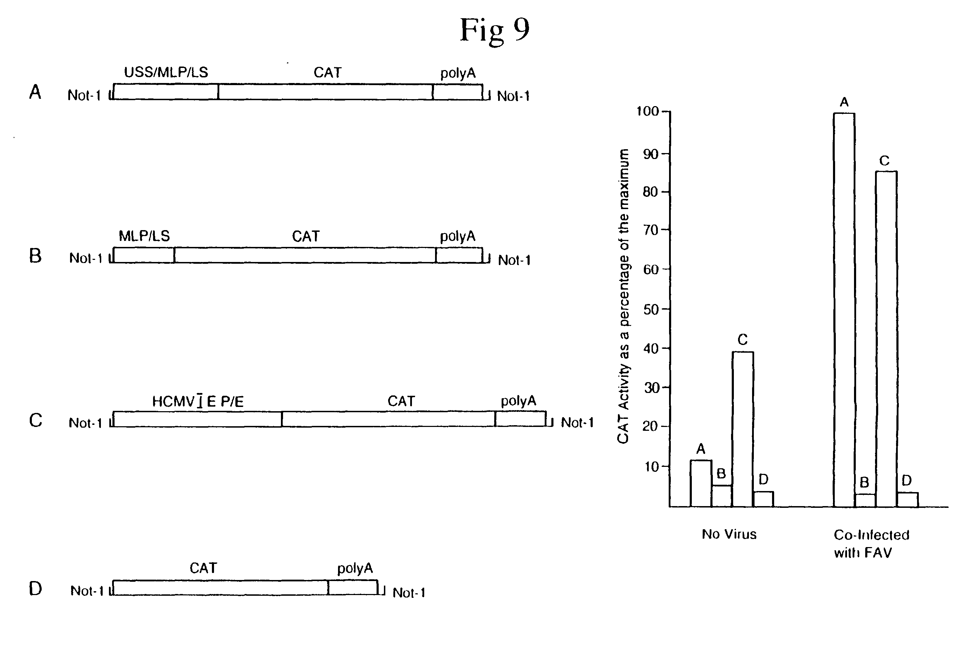

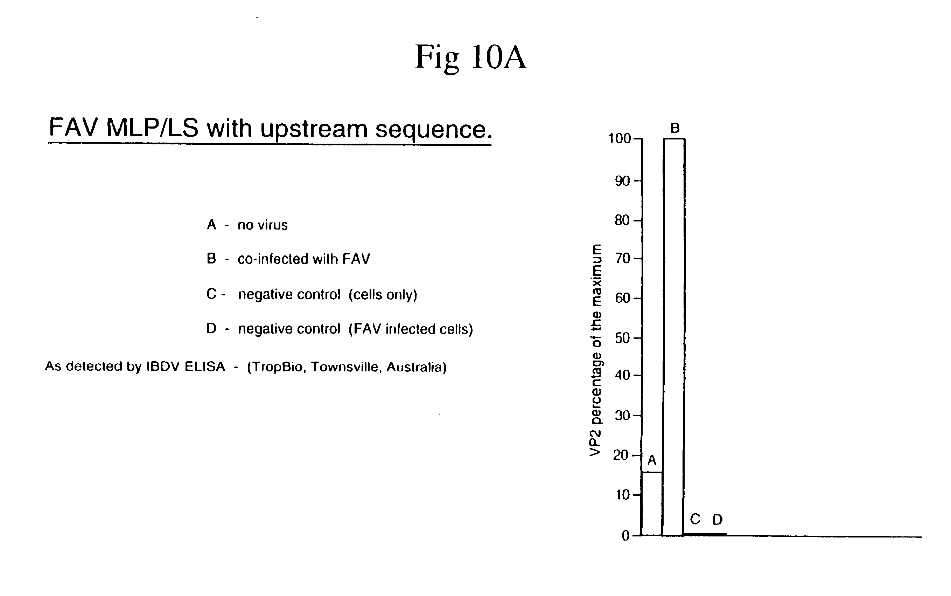

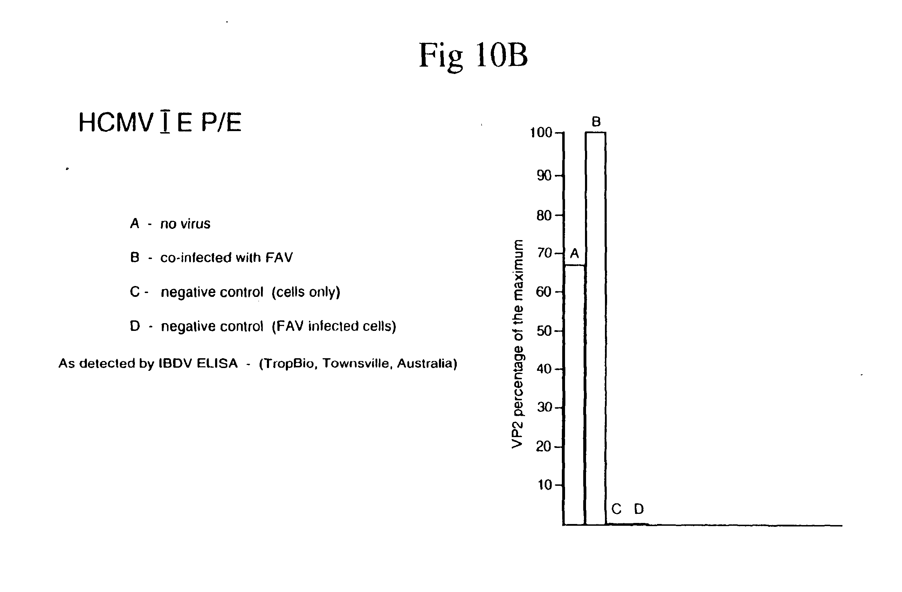

Figures 9 and 10 illustrate the use of expression cassettes of FAV to express foreign genes. Various constructions containing either the FAV MLP/LS with or without upstream sequences (USS), a transcriptional start site, multiple cloning site and a poly A recognition sequence were tested for expression of the chloramphenicol acetylase (CAT) gene or the vp2 antigen gene from infectious bursal disease virus (Azad, A.A., Fahey, K.J., Barrett, S.A., Erny, K.M. and Hudson, P.J. (1986) Expression in Escherichia coli of cDNA Fragments encoding the gene for the host protective antigen of infectious bursal disease virus. Virology 149 190-198). A comparison is made with an expression cassette containing the human cytomegalovirus immediate-early promoter enhancer. The figures show that the FAV MLP/LS cassette when coinfected with whole FAV virus leads to measurable levels of CAT activity or vp2 antigen. These studies would indicate that expression cassettes of the type described here will form the basis of recombinant fowl adenovirus capable of expressing a variety of foreign genes including important vaccine antigens, as previously outlined (page 7) and other agents of commercial benefit to the poultry industry.

### EXAMPLE OF VACCINATION STRATEGY

### 1. Administration of Vaccine comprising one vector

In these experiments day old chicks were used to represent immuno-incompetent birds and 3 week old chickens as representative of chickens approaching immunocompetency. Day-old chicks were infected with CFA20 by aerosol spray. Virus was suspended in sterile isotonic saline at a dose of 5x10⁷/chicken. The birds were placed into a confined area and the virus sprayed into the air at the head height of the birds such that the spray contacted the eyes and beaks of the chickens. The coarse spray was delivered using a pump-action plastic spray bottle. The onset of serum antibody responses were monitored by ELISA and virus neutralization assays. The detailed results are shown in Table 2.

**Table 2. Serum antibody response and virus clearance in day-old chickens inoculated by aerosol with the FAV CFA20**

| Days post-inoculation | Neutralization litre (CFA20)^{a} | ELISA titre recovery^{b} | Virus recovery^{c} |
|---|---|---|---|
| 0 | 0 | 0 | - |
| 7 | 0 | 0 | + |
| 14 | 0 | 0 | + |
| 21 | 0 | 0 | + |
| 28 | 0 | 125 | + |
| | 0 | 70 | - |
| | 0 | 240 | - |
| 35 | 0 | 160 | - |
| | 0 | 300 | - |
| | 0 | 400 | - |
| 42^{d} | 0 | 160 | - |
| | 0 | 100 | - |
| | 0 | 200 | - |
| 47 | 0 | 220 | - |
| | 0 | 110 | - |
| | 0 | 280 | - |
| 54 | 0 | 100 | - |
| | 0 | 70 | - |
| a - reciprocal of virus neutralization titre in serum against CFA20 | | | |
| b - reciprocal of ELISA titre against CFA20 | | | |
| c - virus recovery from the caecal tonsils of 3 chickens at each time point | | | |
| d - re-inoculate with CFA20 | | | |

Virus was recovered from caecal tonsils for 4 weeks post-infection, the disappearance of virus coinciding with the development of circulating antibody which could be detected by ELISA but not by the virus neutralization assay. No virus neutralizing antibodies were detected over the 7 week period of this experiment. However, attempts to re-infect these chicks with CFA20 at 6 weeks after the primary infection were unsuccessful as reflected by the failure to recover virus and the absence of an anamnestic ELISA antibody response in the serum.

When chickens were infected at 3 weeks of age virus could be recovered for only 1-2 weeks postinfection, and again the clearance of virus coincided with development of antibodies detectable by ELISA (Table 3).

**Table 3. Serum antibody response and virus clearance in chickens inoculated by aerosol at 3 weeks of age with the FAV CFA20**

| Days post-inoculation | Neutralization titre (CFA20)^{a} | ELISA titre^{b} CFA20 | Virus recovery^{c} |
|---|---|---|---|
| 0 | 0 | 0 | - |
| 7 | 0 | 0 | + |
| 14 | 0 | 260 | - |
| | 0 | 400 | - |
| | 0 | 440 | - |
| 21 | 0 | 120 | - |
| | 0 | 220 | - |
| | 0 | 200 | - |
| | 0 | 140 | - |
| | 40 | 240 | - |
| 35 | 40 | 100 | - |
| | 80 | 280 | - |
| | 160 | 280 | - |
| 42 | 640 | 560 | - |
| | 80 | 140 | - |
| | 160 | 240 | - |
| a - reciprocal of virus neutralization titre in serum against CFA20 | | | |
| b - reciprocal of ELISA titre against CFA20 | | | |
| c - recovery of virus from the caecal tonsils of 3 chickens at each time point | | | |

Virus neutralizing antibodies could also be detected in the serum of these chickens but not before 4 weeks post-infection (7 weeks of age). This was 2 weeks after the clearance of virus from the caecal tonsils.

### 2. Administration of Consecutive Vaccines

Two combinations were employed for investigating the feasibility of consecutive vaccination by aerosol. In the trial the initial vaccination was with CFA19 (serotype 9) and this was followed later with CFA20. The results are shown in Table 4.

**Table 4. Consecutive vaccination: Serum antibody response of 3 week old chickens infected by aerosol with the FAV CFA19, and re-inoculated with FAV CFA20.**

| Days post-inoculation | Neutralization CFA19 | titre^{a} CFA20 | Virus^{b} re-isolation |
|---|---|---|---|
| 0^{c} | 0 | 0 | - |
| 3 | 0 | 0 | - |
| 7 | 20 | 0 | + |
| | 20 | 0 | + |
| | 20 | 0 | + |
| 14 | 640 | 0 | - |
| | >1280 | 0 | - |
| | >1280 | 0 | - |
| 28^{d} | >1280 | 0 | - |
| | >1280 | 0 | - |
| | >1280 | 0 | - |
| 31 | 2400 | 80 | + |
| | 2400 | 40 | + |
| | 2400 | 80 | + |
| 35 | 2400 | 160 | + |
| | 1200 | 60 | + |
| 42 | >2400 | 320 | - |
| | >2400 | 320 | - |
| a- reciprocal virus neutralization titre against either CFA20 or CFA19 | | | |
| b- virus re-isolation from caecal tonsils | | | |
| c- inoculate by aerosol with CFA19 | | | |
| d- inoculate by aerosol with CFA20 | | | |

Vaccination with CFA19 did not protect against infection with CFA20, (Table 4). Challenge with CFA20 4 weeks after vaccination with CFA19 resulted in the production of CFA20 specific virus neutralizing antibodies and CFA20 virus could be recovered for at least 7 days after challenge. No cross-neutralization between CFA19 and CFA20 was found by the *in vitro* neutralization assays.

The identity of viruses recovered from these chickens was confirmed by restriction endonuclease analysis of their DNA. This demonstrated that only CFA20 was recovered after challenge of CFA19 vaccinated chickens.

### 3. Administration of Simultaneous Vaccines

Simultaneous vaccination of chickens with CFA20 and CFA19 by aerosol was also investigated. The results given in Table 5 show that neither the timing or magnitude of the antibody responses to either of these viruses was compromised by the presence of a second FAV infection.

**Table 5. Simultaneous vaccinations : Serum antibody response of 3 week old chickens inoculated simultaneously by aerosol with the two FAV CFA20 and CFA19**

| Days post-infection | Neutralization titre^{a} | | Virus^{b} re-isolation |
|---|---|---|---|
| | CFA19 | CFA20 | |
| 0^{c} | 0 | 0 | - |
| 3 | 0 | 0 | + |
| 7 | 0 | 0 | + |
| | 0 | 0 | + |
| | 0 | 0 | + |
| 14 | 1280 | 0 | - |
| | 1280 | 0 | - |
| | 1280 | 0 | - |
| 28 | 2400 | 20 | - |
| | 1280 | 40 | - |
| | 2400 | 20 | - |
| 35 | 4800 | 1280 | - |
| | 4800 | 320 | - |
| | 9600 | 320 | - |
| 42 | >20000 | 1280 | - |
| | >20000 | 320 | - |
| a- reciprocal of virus neutralization titre against either CFA19 or CFA20 | | | |
| b- virus re-isolated from caecal tonsils | | | |
| c- chickens inoculated with CFA19 and CFA20 | | | |

The virus neutralizing antibody response to CFA19 was first detected at 14 days post-infection and that to CFA20 at 28 days as found when these viruses were administered alone (Table 3). Restriction enzyme analysis of the recovered viruses showed that at 3 days post-infection only CFA20 could be isolated from the caecal tonsils, however by 7 days both viruses were recovered.

### 4. Induction of an antibody response to a foreign antigen delivered by a recombinant FAV

Chickens were vaccinated with a recombinant fowl adenovirus carrying the gene for VP2 of infectious bursal disease virus (IBDV) or with CFA20. Birds were bled at 0 and 14 days and then weekly after the intraperitoneal administration of the vaccine at one day old. Sera were collected and tested for the presence of antibody against VP2 of IBDV and adenovirus by ELISA. The results in Table 6 show that antibody against VP2 was first detected in the group vaccinated with the recombinant, 14 days after vaccination and peaked at 28 days post-vaccination.

**Table 6. Serum antibody response (reciprocal titre) in chickens given an FAV-VP2 recombinant or CFA20.**

| Vaccine | Antibody | Time post-vaccination (days) | | | | | |
|---|---|---|---|---|---|---|---|
| | | 0 | 14 | 21 | 28 | 35 | 42 |
| FAV-VP2 | VP2 | <100 | >400 | 400 | 1600 | 400 | ND |
| FAV-VP2 | FAV | <100 | 100 | 100 | 400 | 400 | 200 |
| CFA20 | VP2 | <100 | <100 | <100 | <100 | <100 | <100 |
| CFA20 | FAV | <100 | 100 | 100 | 200 | 800 | 800 |

| | | | | | | | |
|---|---|---|---|---|---|---|---|
| ND = not done | | | | | | | |

The birds vaccinated with CFA20 did not have detectable antibody against VP2. Anti-adenovirus antibody was detected at 14 days post vaccination in both groups.

### 5. Induction of protection by the Administration of a recombinant vaccine

Chickens were vaccinated intravenously either with the recombinant adenovirus carrying the IBDV VP2 gene or with the CFA20. Both groups were challenged with virulent infectious bursal disease virus 21 days after vaccination. All birds were killed 4 days later and IBDV in the bursa of Fabricious determined by ELISA. The results in Table 7 show that birds which received the recombinant vaccine were all protected against the infection with viral antigen titres in the bursa of less than ¼. In contrast all the birds immunised with the parental adenovirus had antigen titres of greater than ¹/₁₂₈.

**Table 7. Protection against IBDV induced by the administration of an FAV-VP2 or CFA20.**

| Vaccine | Infected/Total* |
|---|---|
| Recombinant FAV-VP2 | 0/10 |
| FAV CFA20 | 10/10 |

| | |
|---|---|
| * Birds which received the recombinant vaccine all had antigen titres of less than ¼. Birds which received FAV all had titres of greater than ¹/₁₂₈. | |

Although the foregoing specification refers specifically to fowl adenovirus vectors of certain serotype (4, 9 and 10), it will be understood that live infectious avian adenovirus of any type may be employed in this invention. Similarly for the purposes of construction of a FAV vector eukaryotic promoter and leader sequences may be substituted, as well as mutants and variants of the specific sequences mentioned herein. It will be understood that immunization against a variety of diseases, and of a variety of different bird species is encompassed within the scope of this invention despite the fact that the invention has been exemplified only with respect to fowl and a heterologous sequence derived from infectious bursal disease virus. Other birds in which the vector may be effective include turkeys, ducks, pheasants, quail and geese.

## Claims

1. A recombinant avian adenovirus vector incorporating, and capable of expression of, at least one heterologous nucleotide sequence, wherein the at least one heterologous nucleotide sequence is inserted into a non-essential region at the right terminal end of the genome of the avian adenovirus.

2. A recombinant avian adenovirus according to claim 1 wherein said right terminal end is located at map units 92-100.

3. A recombinant avian adenovirus vector as claimed in claim 6, wherein the at least one heterologous nucleotide sequence is inserted into a non-essential region of the genome located at the right end of the genome at map units 97 to 99.9.

4. A recombinant vector as claimed in claim 1 wherein said recombinant avian adenovirus comprises a live avian adenovirus having virion structural proteins unchanged from those in a native avian adenovirus from which said recombinant avian adenovirus is derived.

5. A recombinant vector as claimed in any one of claims 1 to 4 wherein said at least one heterologous nucleotide sequence is capable of expression as an antigenic polypeptide.

6. A recombinant vector as claimed in any one of claims 1 to 5 wherein said heterologous nucleotide sequence encodes an antigenic determinant of infectious bursal disease virus.

7. A recombinant vector as claimed in any one of claims 1 to 6 wherein said recombinant avian adenovirus is selected from the group consisting of serotypes 4, 9 and 10.

8. A recombinant avian adenovirus vector as claimed in any one of the claims, wherein the at least one heterologous nucleotide sequence encodes an immunopotentiator or an antigenic determinant of an avian infectious disease.

9. A recombinant avian adenovirus vector as claimed in claim 8, wherein the avian infectious disease is selected from: intestinal infections caused by parasites; respiratory viral infections; infections of internal organs; Newcastle Disease; Marek's Disease; Egg Drop Syndrome; Inclusion Body Hepatitis; Infectious Laryngotracheitis; Mycoplasma; Chicken Anemia; Avian Influenza; Coccidia; Infectious Bronchitis; Infections Bursal Disease; and Avian Encephalomyelitis.

10. A recombinant avian adenovirus vector as claimed in claim 8, wherein said immunopotentiator is selected from cytokines and growth promoters.

11. A recombinant avian adenovirus vector as claimed in claim 8, wherein said immunopotentiator is selected from chicken myelomonocytic growth factor and insulin like growth factors.

12. A recombinant avian adenovirus vector as claimed in any one of the preceding claims, wherein said recombinant avian adenovirus is a fowl adenovirus.

13. A recombinant avian adenovirus as claimed in any one of the preceding claims, for use as a medicament.

14. Use, in the preparation of a medicament, of a recombinant avian adenovirus as claimed in any one of the preceding claims.

15. Use as claimed in claim 14, wherein the medicament is a medicament for birds.

16. Use as claimed in claim 14, wherein the medicament is a medicament for chickens.

17. A method of producing a recombinant avian adenovirus vector for use as a vaccine comprising inserting into a non-essential region at the right terminal end of an avian adenovirus genome, at least one heterologous nucleotide sequence in association with an effective promoter sequence.

18. A method as claimed in claim 17 wherein prior to insertion of said heterologous nucleotide sequence, a restriction enzyme site is inserted into said non-essential region of said avian adenovirus genome.

19. A method as claimed in claim 17 or claim 18, wherein the recombinant avian adenovirus is as defined in any one of claims 1 to 13.

20. A recombinant vaccine for generating and/or optimising antibodies or cell mediated immunity so as to provide or enhance protection against infection by an infectious organism in birds, said vaccine comprising at least one recombinant avian adenovirus vector incorporating, and capable of expression of at least one heterologous nucleotide sequence, the said heterologous nucleotide sequence being inserted into a non-essential region at the right terminal end of the genome of the avian adenovirus, and suitable carriers and/or excipients.

21. A recombinant vaccine as claimed in claim 20 wherein said carriers and/or excipients are selected such that said vaccine is deliverable in the form of an aerosol spray.

22. A recombinant vaccine as claimed in claim 20 or claim 21. wherein the recombinant avian adenovirus is as defined in any one of claims 1 to 13.

## Patentansprüche

1. Rekombinanter Vogel-Adenovirusvektor, der mindestens eine heterologe Nukleotidsequenz einschließt und in der Lage ist, sie zu exprimieren, **dadurch gekennzeichnet, dass** die mindestens eine heterologe Nukleotidsequenz in eine nicht essentielle Region an dem rechten terminalen Ende des Genoms des Vogel-Adenovirus eingefügt ist.

2. Rekombinanter Vogel-Adenovirusvektor nach Anspruch 1, **dadurch gekennzeichnet, dass** das rechte terminale Ende an Karteneinheiten 92 - 100 lokalisiert ist.

3. Rekombinanter Vogel-Adenovirusvektor wie in Anspruch 2 beansprucht, **dadurch gekennzeichnet, dass** die mindestens eine heterologe Nukleotidsequenz in eine nicht essentielle Region des Genoms eingefügt ist, die am rechten Ende des- Genoms bei Karteneinheiten 97 - 99,9 eingefügt ist.

4. Rekombinanter Vogel-Adenovirusvektor wie in Anspruch 1 beansprucht, **dadurch gekennzeichnet, dass** das rekombinante Vogel-Adenovirus einen lebendigen Vogel-Adenovirus umfasst, der strukturelle Virion-Proteine aufweist, die gegenüber denen in einem nativen Vogel-Adenovirus, von dem das rekombinante Vogel-Adenovirus abgeleitet ist, nicht verändert sind.

5. Rekombinanter Vektor wie in einem der Ansprüche 1 bis 4 beansprucht, **dadurch gekennzeichnet, dass** die mindestens eine heterologe Nukleotidsequenz in der Lage ist, als antigenes Polypeptid exprimiert zu werden.

6. Rekombinanter Vektor wie in einem der Ansprüche 1 bis 5 beansprucht, **dadurch gekennzeichnet, dass** die heterologe Nukleotidsequenz eine antigene Determinante von infektiösem Bursitis-Virus kodiert.

7. Rekombinanter Vektor wie in einem der Ansprüche 1 bis 6 beansprucht, **dadurch gekennzeichnet, dass** das rekombinante Vogel-Adenovirus ausgewählt ist aus der Gruppe bestehend aus Serotypen 4, 9 und 10.

8. Rekombinanter Vogel-Adenovirusvektor wie in einem der vorherigen Ansprüche beansprucht, **dadurch gekennzeichnet, dass** die mindestens eine heterologe Nukleotidsequenz für einen Immunpotentiator oder eine antigene Determinante einer infektiösen Vogelerkrankung kodiert.

9. Rekombinanter Vogel-Adenovirusvektor wie in Anspruch 8 beansprucht, **dadurch gekennzeichnet, dass** die infektiöse Vogelerkrankung ausgewählt ist aus: von Parasiten verursachten Darminfektionen; virale Atemwegserkrankungen, Infektionen innerer Organe; Newcastle-Krankheit; Marek'sche Krankheit; Egg Drop-Syndrom; Inclusion body-Hepatitis; infektiöse Laryngotracheitis; Mykoplasmen ; Hühneranämie; Vogelinfluenza; Coccidia; infektiöse Bronchitis; infektiöse Bursitis- und Vogelencephalomyelitis.

10. Rekombinanter Vogel-Adenovirusvektor wie in Anspruch 8 beansprucht, **dadurch gekennzeichnet, dass** der Immunpotentiator aus Cytokinen und Wachstumspromotoren ausgewählt ist.

11. Rekombinanter Vogel-Adenovirusvektor wie in Anspruch 8 beansprucht, **dadurch gekennzeichnet, dass** der Immunpotentiator aus Huhn Myelomonozytischem Wachstumsfaktor und insulinartigen Wachstumsfaktoren ausgewählt ist.

12. Rekombinanter Vogel-Adenovirusvektor, wie in einem der vorangehenden Ansprüche beansprucht, **dadurch gekennzeichnet, dass** der rekombinante Vogel-Adenovirus ein Geflügel-Adenovirus ist.

13. Rekombinanter Vogel-Adenovirusvektor wie in einem der vorangehenden Ansprüche beansprucht zur Verwendung als Medikament.

14. Verwendung von einem rekombinanten Vogel-Adenovirus, wie in einem der vorangehenden Ansprüche beansprucht, zur Herstellung eines Medikaments.

15. Verwendung wie in Anspruch 14 beansprucht, **dadurch gekennzeichnet, dass** das Medikament ein Medikament für Vögel ist.

16. Verwendung wie in Anspruch 14 beansprucht, **dadurch gekennzeichnet, dass** das Medikament ein Medikament für Hühner ist.

17. Verfahren zur Herstellung eines rekombinanten Vogel-Adenovirusvektors zur Verwendung als Impfstoff, Schritte umfassend, bei denen man in eine nicht essentielle Region an dem rechten terminalen Ende eines Vogel-Adenovirusgenoms mindestens eine heterologe Nukleotidsequenz in Verbindung mit einer effektiven Promotersequenz einfügt.

18. Verfahren wie in Anspruch 17 beansprucht, **dadurch gekennzeichnet, dass** man vor dem Einfügen der heterologen Nukleotidsequenz eine Restriktionsenzym-Stelle in die nicht essentielle Region des Vogel-Adenovirusgenoms einfügt.

19. Verfahren wie in Anspruch 17 oder in Anspruch 18 beansprucht, **dadurch gekennzeichnet, dass** das rekombinante Vogel-Adenovirus wie in einem der Ansprüche 1 bis 13 definiert ist.

20. Rekombinanter Impfstoff zur Herstellung und/oder Optimierung von Antikörpern oder zellvermittelter Immunität zur Bereitstellung oder Verbesserung von Schutz gegen Infektion durch einen infektiösen Organismus bei Vögeln, wobei der Impfstoff mindestens einen rekombinanten Vogel-Adenovirusvektor, der mindestens eine heterologe Nukleotidsequenz einschließt und in der Lage ist, sie zu exprimieren, **dadurch gekennzeichnet, dass** die heterologe Nukleotidsequenz in eine nicht essentielle Region an dem rechten terminalen Ende des Genoms des Vogel-Adenovirus eingefügt ist, und geeignete Träger und/oder Hilfsstoffe umfasst.

21. Rekombinanter Impfstoff wie in Anspruch 20 beansprucht, **dadurch gekennzeichnet, dass** die Träger und/oder Hilfstoffe so ausgewählt sind, dass der Impfstoff in der Form eines Aerosol-Sprays verabreichbar ist.

22. Rekombinanter Impfstoff wie in Anspruch 20 oder Anspruch 21 beansprucht, **dadurch gekennzeichnet, dass** das rekombinante Vogel-Adenovirus wie in einem der Ansprüche 1 bis 13 definiert ist.

## Revendications

1. Vecteur adénovirus aviaire recombinant incorporant, et capable d'exprimer, au moins une séquence nucléotidique hétérologue, dans lequel la au moins une séquence nucléotidique hétérologue est insérée dans une région non essentielle au niveau de l'extrémité terminale droite du génome de l'adénovirus aviaire.

2. Vecteur adénovirus aviaire recombinant selon la revendication 1 dans lequel ladite extrémité terminale droite est située aux unités cartographiques 92 à 100.

3. Vecteur adénovirus aviaire recombinant selon la revendication 6 dans lequel la au moins une séquence nucléotidique hétérologue est insérée dans une région non essentielle du génome située au niveau de l'extrémité terminale droite du génome aux unités cartographiques 97 à 99,9.

4. Vecteur recombinant selon la revendication 1 dans lequel ledit adénovirus aviaire recombinant comprend un adénovirus aviaire vivant dont le virion est constitué de protéines structurelles inchangées par rapport à celles d'un adénovirus aviaire natif à partir duquel ledit adénovirus aviaire recombinant est dérivé.

5. -Vecteur recombinant selon l'une quelconque des revendications. 1 à 4 dans lequel ladite séquence nucléotidique hétérologue est capable d'être exprimée comme un polypeptide antigénique.

6. Vecteur recombinant selon l'une quelconque des revendications 1 à 5 dans lequel ladite séquence nucléotidique hétérologue code pour un déterminant antigénique-du virus de la bursite infectieuse.

7. Vecteur recombinant selon l'une quelconque des revendications 1 à 6 dans lequel ledit adénovirus aviaire recombinant est sélectionné dans le groupe constitué des sérotypes 4, 9 et 10.

8. Vecteur adénovirus aviaire recombinant selon l'une quelconque des revendications précédentes dans lequel la séquence nucléotidique hétérologue code un immunopotentialisateur ou un déterminant antigénique d'une pathologie infectieuse aviaire.

9. Vecteur adénovirus aviaire recombinant selon la revendication 8 dans lequel la pathologie aviaire est sélectionnée entre : infections intestinales causées par des parasites, infections respiratoires virales, infections des organes internes, maladie-de Newcastle, maladie de Marek, syndrome des chutes de ponte, hépatite du corps d'inclusion, laryngotrachéite infectieuse, mycoplasme, anémie du poulet, grippe aviaire, coccidies, bronchite infectieuse, bursite infectieuse et encéphalomyélite aviaire.

10. Vecteur adénovirus aviaire recombinant-selon la revendication 8, dans lequel ledit immunopotentialisateur est sélectionné parmi les cytokines ou les facteurs -de croissance.

11. Vecteur adénovirus aviaire recombinant selon la revendication 8, dans lequel ledit immunopotentialisateur est sélectionné entre le facteur de croissance myélomonocytaire de poulet et les facteurs-de croissance du type insuline.

12. Vecteur adénovirus aviaire recombinant selon l'une quelconque des -revendications précédentes, dans-lequel ledit adénovirus de recombinaison avien est un adénovirus de volaille.

13. Vecteur adénovirus aviaire recombinant- selon l'une quelconque des revendications précédentes, destiné à être utilisé-dans un médicament.

14. Utilisation dans la préparation d'un médicament d'un adénovirus aviaire recombinant selon l'une quelconque des revendications précédentes.

15. Utilisation selon la revendication 14, dans laquelle le médicament est destiné aux oiseaux.

16. Utilisation selon la revendication 14, dans laquelle le médicament est destiné aux poulets.

17. Procédé de production d'un vecteur adénovirus aviaire recombinant destiné à être utilisé dans un vaccin, comprenant l'insertion dans une région non essentielle à l'extrémité terminale droite d'un génome d'adénovirus aviaire d'au moins une séquence nucléotidique hétérologue en association avec une séquence promoteur efficace.

18. Procédé selon la revendication 17 dans lequel avant l'insertion de ladite séquence nucléotidique hétérologue, un site d'enzymes de restriction est inséré dans ladite région non essentielle dudit génome d'adénovirus aviaire.

19. Procédé selon la revendication 17 ou 18, dans lequel l'adénovirus aviaire recombinant est tel que défini dans l'une quelconque des revendications 1 à 13.

20. Vaccin recombinant pour générer et/ou optimiser l'immunité assurée par les anticorps ou les cellules de manière à assurer ou à améliorer la protection contre une infection provoquée par un organisme infectieux chez les oiseaux, ledit vaccin comprenant au moins un vecteur adénovirus aviaire recombinant, et étant capable d'exprimer au moins une séquence nucléotidique hétérologue, ladite séquence nucléotidique hétérologue étant insérée dans une région non essentielle à l'extrémité terminale droite du génome de l'adénovirus aviaire, et des transporteurs et/ou excipients appropriés.

21. Vaccin recombinant selon la revendication 20 dans lequel lesdits transporteurs et/ou excipients sont sélectionnés en sorte que le vaccin peut être administré sous forme d'un aérosol.

22. Vaccin recombinant selon la revendication 20 ou 21 dans lequel l'adénovirus aviaire recombinant est tel que défini dans l'une quelconque des revendications 1 à 13.
